# EUROPÄISCHE PATENTSCHRIFT

(11) **EP 0 624 341 B1**
(45) Veröffentlichungstag und Bekanntmachung des Hinweises auf die Patenterteilung: **22.07.1998**
(21) Anmeldenummer: 94104764.9
(22) Anmeldetag: 25.03.1994
(51) Int. Cl.: A61B 5/22, A61B 5/00, A61B 5/05, A63B 21/00

(54) **Heimtrainer mit Bio-Feedback**
Hometrainer with bio-feedback
Home-trainer avec bio-contre réaction

(30) Priorität: 14.05.1993 DE 9307352 U
(43) Veröffentlichungstag der Anmeldung: 17.11.1994
(73) Patentinhaber: DAUM ELECTRONIC GmbH, D-90587 Veitsbronn (DE)
(72) Erfinder: Daum, Wilhelm, D-90768 Vach (DE)
(74) Vertreter: Rau, Manfred, Dr. Dipl.-Ing.

(56) Entgegenhaltungen:
- EP-A- 0 235 312
- WO-A-89/00064
- DE-A- 3 301 550
- DE-U- 9 109 354
- US-A- 4 591 729

## Beschreibung

Die Erfindung richtet sich auf einen Heimtrainer, insbesondere in Form eines Standfahrrads, wobei an dem Heimtrainer ein Mikroprozessor und eine Einrichtung zur Erfassung der von der trainierenden Person erbrachten Leistung sowie ein Sensor zur Messung des Durchgangswiderstandes der Hautoberfläche vorgesehen ist, welcher mit einem Mikroprozessor verbunden ist, wobei der Mikroprozessor seinerseits in Abhängigkeit von dem aus dem Sensor erhaltenden Signal akustische und/oder optische Anzeigegeräte steuert. Ein solcher Heimtrainer ist aus EP-A-0 235 312 bekannt.

Die Bedeutung körperlicher Fitness hat im Bewußtsein einer breiten Öffentlichkeit in jüngerer Zeit erhebliche Bedeutung erlangt. In diesem Zusammenhang sind auch zahlreiche Heimtrainer, z.B. in Form von Rudergeräten insbesondere aber in Form von Standfahrrädern entwickelt worden, welche es nicht nur ermöglichen, zuhause eine zeitlich und in ihrer Intensität einstellbare körperliche Trainingsleistung zu erbringen sondern welche auch mit Zusatzeinrichtungen ausgestattet worden sind, um wichtige körperliche Kenndaten über den Benutzer zu sammeln und dementsprechend Informationen über den körperlichen Zustand und Trainingsfortschritt zu geben um dem Trainierenden einerseits ein Erfolgserlebnis durch einen erkennbaren meßbaren Trainingsfortschritt zu geben und andererseits auch einen sportmedizinisch optimalen Trainingsaufbau zu ermöglichen.

Als Maßstab für die körperliche Beanspruchung und die Erholungsrate nach Beendigung der körperlichen Beanspruchung wurde insbesondere die Pulsfrequenz der trainierenden Person gemessen. Ein derartiges Gerät ist beispielsweise in dem britischen Patent 1 593 839 beschrieben. Die US-PS 4 367 752 beschreibt eine Einrichtung zur Erfassung der Herzaktivität mit einer visuellen Anzeigeeinrichtung, welche insbesondere für das Training von Läufern im Freien bestimmt ist.

Die DE-OS 39 08 756 beschreibt eine Speichereinheit zur Steuerung eines Ergometers, welche die Datenaufnahme während des Trainings ermöglicht. Auch die DE-AS 26 30 293 beschreibt ein medizinisches Gerät mit einem Pulsaufnehmer, welches eine Auswertung der Trainingsleistung unter Berücksichtigung spezifischer Kenndaten der trainierenden Person ermöglicht. Ein Standfahrrad mit Pulsmeßgerät und Datenverarbeitung zur Optimierung des Trainingsaufbaus wird in der US-PS 4 911 427 beschrieben. Auch die DE-OS 36 01 054 und die DE-OS 41 07 323 beschreiben Geräte, die unter Berücksichtigung der Pulsaktivität eine Dosierung der körperlichen Belastung und eine Ausrichtung des Trainings ermöglichen.

Die bekannten sportmedizinisch ausgerichteten Geräte setzen voraus, daß die trainierende Person sich sehr intensiv geistig mit den jeweiligen Anzeigen und Trainingsmethoden auseinandersetzt und nicht nur erkennt, was jeweils hinsichtlich des Trainings zu veranlassen ist, sondern auch die Willenskraft zur Durchführung des Erforderlichen aufbringt.

Tatsächlich zeigt die praktische Erfahrung, daß zwar sehr viele Personen mit dem guten Vorsatz das Training mit derartigen Geräten aufnehmen, die von den Geräten gebotenen Möglichkeiten zu nutzen daß jedoch das Training häufig deshalb wieder abgebrochen wird, weil der psychologische Anreiz relativ gering ist, sich ohne Ansprache und irgendwie reizvolle Reaktion allein mit einem derartigen Gerät auseinanderzusetzen.

Hiervon ausgehend liegt der Erfindung die Aufgabe zugrunde, einen Heimtrainer der eingangs genannten Art so auszubilden, daß unter Beibehaltung der Möglichkeit eines sportmedizinisch sinnvollen Trainings der psychologische Anreiz zur Benutzung auch über längere Zeiträume erhöht wird und auch die Zeit nach der aktiven körperlichen Betätigung einbezogen wird.

Diese Aufgabe wird erfindungsgemäß dadurch gelöst, daß ein akustisches Anzeigegerät einen Synthesizer umfaßt, der eine Melodie erzeugt, deren Abspielgeschwindigkeit und/oder Tonlage in Abhängigkeit von dem Abklingen des von den Sensoren ermittelten Erregungszustandes langsamer bzw. tiefer wird.

Bei einer weiteren bevorzugten Ausführungsform ist vorgesehen, daß zusätzlich oder alternativ zu dem Synthesizer ein Kassettenplayer an dem Heimtrainer angeordnet ist, der es ermöglicht, Kassetten einzulegen auf welchen ein Trainingsprogramm derart abgespeichert ist, daß die trainierende Person sprachliche und/oder musikalisch-rhythmische Anweisungen hinsichtlich des Trainingsverhaltens erhält.

Dabei ist es insbesondere möglich, z.B. in Abhängigkeit einer aus dem letzten Training abgespeicherten Fitnessnote, aus einer Mehrzahl abgespeicherter Trainingsprogramme durch den Mikroprozessor solche Programme auswählen zu lassen die dem durch den Mikroprozessor bzw. durch die Sensoren ermittelten tatsächlichen Trainingszustand der trainierenden Person am besten entsprechen.

Statt eines Kassettenplayers kann natürlich auch ein Abspielgerät für Kompakt-Discs oder Mini-Discs verwendet werden.

Nachfolgend wird die Erfindung anhand eines bevorzugten Ausführungsbeispiels in Verbindung mit der Zeichnung näher beschrieben. Dabei zeigen
- Fig. 1: eine schematische Darstellung eines erfindungsgemäßen Heimtrainers in Form eines Standfahrrads und
- Fig. 2: ein schematisches Blockschaltbild.

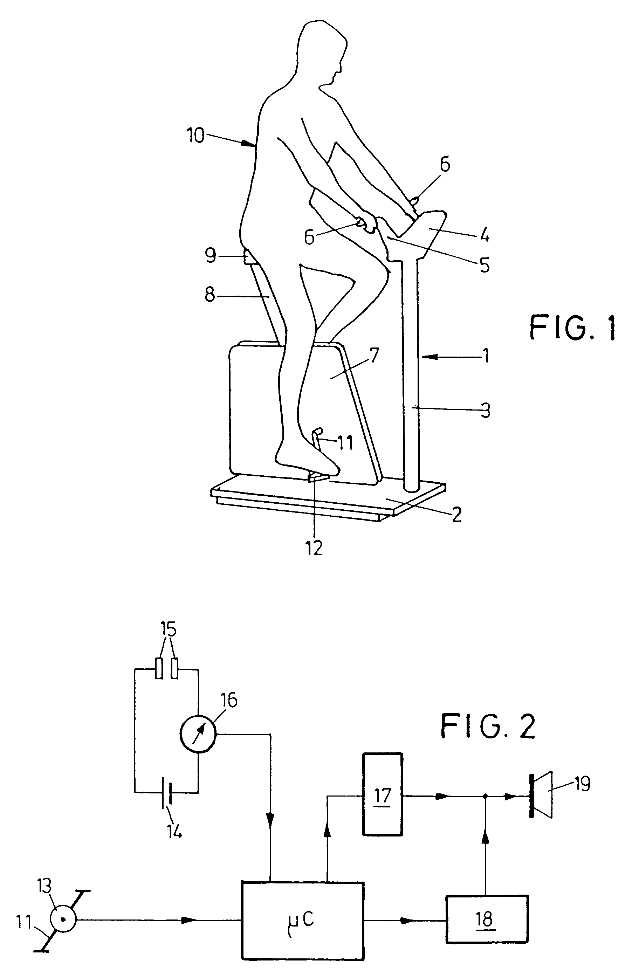

In Fig. 1 ist schematisch ein Standfahrrad 1 dargestellt, welches eine Grundplatte 2 umfaßt, an welcher eine Standsäule 3 angeordnet ist. An der Oberseite der Standsäule 3 ist eine Anzeigetafel 4 vorgesehen. Seitlich derselben erstreckt sich eine lenkerartige Anordnung 5 mit zwei Handgriffen 6.

Hinter der Standsäule 3 ist ein Gehäuse 7 an der Grundplatte 2 angeordnet, von welchem sich eine Sitzsäule 8 mit einem Sitz 9 für die trainierende Person 10 nach oben erstreckt. Weiterhin ist in dem Gehäuse 7 eine Tretkurbelanordnung 11 mit Fußpedalen 12 untergebracht. In an sich bekannter Weise kann in dem Gehäuse 7 auch eine Wirbelstrombremse vorgesehen sein, um den Tretwiderstand einstellbar machen zu können.

Die auf die Pedale 12 von der trainierenden Person 10 übertragene Tretleistung wird durch eine Meßeinrichtung 13 erfaßt und dem Eingang eines Mikrocomputers µC zugeführt. Über eine Spannungsquelle 14, Sensoren 15 und ein Meßgerät 16, dessen Ausgang wiederum mit dem Mikrocomputer µC verbunden ist, wird der sogenannte psychogalvanische Hauteffekt, d.h. der Oberflächenwiderstand der Haut gemessen und dem Mikrocomputer µC zugeführt. Ein Ausgang des Mikrocomputers ist mit einem Synthesizer 17 und ein anderer Ausgang mit einem Kassettenplayer 18 verbunden, deren Ausgänge wiederum an einem Lautsprecher 19 anliegen.

Durch die vorstehend beschriebene Schaltungsanordnung ist es möglich, den Synthesizer 17 während des Trainings oder nach Beendigung der körperlichen Trainingsleistung so anzusteuern, daß eine von ihm generierte Melodie in der Tonlage abgesenkt und/oder in der Geschwindigkeit verlangsamt wird, wenn der psychogalvanische Hauteffekt abklingt, d.h. es findet ein Feedback statt, wodurch das Abklingen des trainingsbedingten Erregungszustandes gefördert wird, und die trainierende Person erhält einen akustischen Anreiz.

Außerdem ist es möglich, über den Kassettenplayer 18, ausgewählt durch den Mikrocomputer µC, durch einen Sprecher und/oder musikalisch-rhythmische Trainingsmaßnahmen vorzugeben, so daß die trainierende Person mit ihrem Trainingseifer nicht alleine gelassen wird, sondern sich motiviert und angeleitet fühlt.

## Patentansprüche

1. Heimtrainer, insbesondere in Form eines Standfahrrads, wobei an dem Heimtrainer ein Mikroprozessor und eine Einrichtung zur Erfassung der von der trainierenden Person erbrachten Leistung sowie ein Sensor zur Messung des Durchgangswiderstandes der Hautoberfläche vorgesehen ist, welcher mit einem Mikroprozessor verbunden ist, wobei der Mikroprozessor seinerseits in Abhängigkeit von dem aus dem Sensor erhaltenden Signal akustische und/oder optische Anzeigegeräte steuert, dadurch gekennzeichnet, daß ein akustisches Anzeigegerät einen Synthesizer (17) umfaßt, der eine Melodie erzeugt, deren Abspielgeschwindigkeit und/oder Tonlage in Abhängigkeit von dem Abklingen des von dem Sensor (15) ermittelten Erregungszustandes langsamer und/oder tiefer wird.

2. Heimtrainer, insbesondere nach Anspruch 1, dadurch gekennzeichnet, daß ein Kassettenplayer bzw. ein Abspielgerät für Kompakt-Discs oder Mini-Discs vorgesehen ist, wobei auf dem jeweiligen Speichermedium ein Trainingsprogramm derart abgespeichert ist, daß die trainierende Person sprachliche Anweisungen hinsichtlich des Trainingsverhaltens erhält.

3. Heimtrainer nach Anspruch 2, dadurch gekennzeichnet, daß der Mikroprozessor (µC) mit dem Kassettenplayer (18) od.dgl. verbunden ist derart, daß der Mikroprozessor in Abhängigkeit von dem Trainingszustand der trainierenden Person, z.B. entsprechend der abgespeicherten Fitnessnote aus dem letzten Trainingsdurchgang, ein geeignetes, abgespeichertes Trainingsprogramm auswählt.

4. Heimtrainer nach Anspruch 2, gekennzeichnet durch eine Unterbrechungs-Schaltungsanordnung zur Unterbrechung der Trainingsanweisungen aus dem Kassettenplayer bzw. dem Abspielgerät immer dann, wenn über den Mikroprozessor festgestellt wird, daß Grenzwerte für den Puls, die theoretisch zurückgelegte Entfernung, die theoretisch gefahrene Geschwindigkeit usw. überschritten werden.

5. Heimtrainer nach Anspruch 1, gekennzeichnet durch wenigstens eine Vergleichstaste zum Abruf der vom letzten Trainingsdurchgang abgespeicherten Trainingswerte und zum Vergleich mit den abgespeicherten Werten des aktuell absolvierten Trainingsdurchgangs.

## Claims

1. A home trainer, in particular in the form of a stationary bicycle, a microprocessor and a device for collecting the performance of a person exercising as well as a sensor for measuring the electric resistance of the skin surface being provided on the home trainer, which sensor is connected with a microprocessor, the microprocessor triggering acoustic and/or optical indicator instruments in dependence on the signal received from the sensor, characterized in that an acoustic indicator instrument comprises a synthesizer (17) generating a melody which slows down and/or gets deeper in pitch in dependence on the subsiding of the state of excitement detected by the sensors (15).

2. A home trainer, in particular according to claim 1, characterized in that a cassette recorder or a reproducer, respectively, of compact disks or mini disks is provided, a training program being stored on the respective storage medium such that the person exercising gets spoken instructions on the behavior for exercising.

3. A home trainer according to claim 2, characterized in that the microprocessor (µC) is connected with the cassette recorder (18) or the like such that the microprocessor selects a suitable stored training program in dependence on the training condition of the person exercising, for instance in accordance with the fitness characteristics stored from the latest exercise.

4. A home trainer according to claim 2, characterized by an interrupt circuit arrangement for interrupting the training instructions from the cassette recorder or the reproducer whenever the microprocessor detects that limits of the pulse rate, of the distance covered theoretically, of the theoretical velocity of driving etc. are exceeded.

5. A home trainer according to claim 1, characterized by at least one reference key for calling the training characteristics stored from the latest training cycle and for comparison with the stored characteristics of the training cycle actually performed.

## Revendications

1. Home-trainer ou dispositif d'entraînement utilisé à domicile, notamment sous la forme d'une bicyclette fixe, dans lequel il est prévu un microprocesseur et un dispositif pour détecter l'énergie produite par la personne qui s'entraîne, ainsi qu'un capteur servant à mesurer la résistance interne de la surface de la peau et qui est relié à un microprocesseur, le microprocesseur commandant pour sa part des appareils indicateurs acoustiques et/ou optiques en fonction du signal reçu du capteur, caractérisé en ce qu'un appareil indicateur acoustique comprend un synthétiseur (17) qui produit une mélodie, dont la vitesse de reproduction et/ou l'intensité acoustique diminuent en fonction de la décroissance de l'état d'excitation déterminé par le capteur (15).

2. Home-trainer ou dispositif d'entraînement utilisé à domicile, notamment selon la revendication 1, caractérisé en ce qu'il est prévu un lecteur de cassettes ou un appareil de lecture de disques compacts ou de minisdisques, un programme d'entraînement étant mémorisé sur le support d'enregistrement considéré de telle sorte que la personne, qui s'entraîne, reçoit des indications parlées concernant le comportement d'entraînement.

3. Home-trainer ou dispositif d'entraînement utilisé à domicile, selon la revendication 2, caractérisé en ce que le microprocesseur (µc) est relié au lecteur de cassettes (17) ou analogue de telle sorte que le microprocesseur sélectionne, en fonction de l'état d'entraînement de la personne qui s'entraîne, par exemple en fonction de la note d'aptitude mémorisée obtenue à partir du dernier cycle d'entraînement, un programme d'entraînement approprié mémorisé.

4. Home-trainer ou dispositif d'entraînement utilisé à domicile, selon la revendication 2, caractérisé par un montage d'interruption servant à interrompre les indications d'entraînement délivrées par le lecteur de cassettes ou l'appareil de lecture chaque fois qu'il est établi, par l'intermédiaire du microprocesseur, que des valeurs limites pour le poule, la distance théoriquement parcourue, la vitesse théorique de déplacement, etc. sont dépassées par valeurs supérieures.

5. Home-trainer ou dispositif d'entraînement utilisé à domicile, selon la revendication 1, caractérisé par au moins une touche de comparaison servant à appeler des valeurs d'entraînement mémorisées lors du dernier cycle d'entraînement et à comparer ces valeurs aux valeurs mémorisées du cycle d'entraînement actuellement exécuté.
